# EUROPEAN PATENT APPLICATION

(11) **EP 4 706 571 A1**
(43) Date of publication of application: **11.03.2026**
(21) Application number: 25196440.9
(22) Date of filing: 18.08.2025
(51) Int. Cl.: A61B 18/14, A61B 18/00

(54) **SYSTEMS AND METHODS FOR ELECTROPORATION DEVICES**

(30) Priority: 05.09.2024 US 202463691012 P
(71) Applicant: St. Jude Medical, Cardiology Division, Inc., St. Paul, MN 55117 (US)
(72) Inventor: HOFFER, Nathaniel J., Champlin, 55316 (US); BELL, Ryan C., Minneapolis, 55414 (US); MOON, Loell Boyce, Ham Lake, 55304 (US)
(74) Representative: Mathys & Squire

(57) **Abstract**

Systems and methods for electroporation catheters are disclosed herein. An electroporation catheter includes a shaft, and a plurality of splines forming a basket around a distal portion of the shaft, wherein each spline of the plurality of splines comprises at least one energizable electrode. The electroporation catheter further includes a balloon positioned within the basket formed by the plurality of splines, the balloon comprising at least one radiopaque marking.

## Description

### Field of the invention

The present disclosure relates generally to tissue ablation systems. In particular, the present disclosure relates to electroporation catheters including catheter assemblies with balloons having at least one radiopaque marking.

### Background

It is generally known that ablation therapy may be used to treat various conditions afflicting the human anatomy. For example, ablation therapy may be used in the treatment of atrial arrhythmias. When tissue is ablated, or at least subjected to ablative energy generated by an ablation generator and delivered by an ablation catheter, lesions form in the tissue. Electrodes mounted on or in ablation catheters are used to create tissue apoptosis in cardiac tissue to correct conditions such as atrial arrhythmia (including, but not limited to, ectopic atrial tachycardia, atrial fibrillation, and atrial flutter).

Electroporation is a non-thermal ablation technique that involves applying strong electric-fields that induce pore formation in the cellular membrane. The electric field may be induced by applying a relatively short duration pulse which may last, for instance, from a nanosecond to several milliseconds. Such a pulse may be repeated to form a pulse train. When such an electric field is applied to tissue in an in vivo setting, the cells in the tissue are subjected to trans-membrane potential, which opens the pores on the cell wall. Electroporation may be reversible (i.e., the temporally-opened pores will reseal) or irreversible (i.e., the pores will remain open). For example, in the field of gene therapy, reversible electroporation (i.e., temporarily open pores) is used to transfect high molecular weight therapeutic vectors into the cells. In other therapeutic applications, a suitably configured pulse train alone may be used to cause cell destruction, for instance by causing irreversible electroporation.

For catheters used to deliver bipolar energy using irreversible electroporation (IRE) or pulsed field ablation (PFA) it is important to ensure that catheter electrodes are close to or contacting a vessel wall. Generally, the closer the electrodes to the vessel wall, the larger the lesion size.

Determining the relative orientation and/or position of catheters in such procedures is therefore desirable, and localization and navigation systems for visualization, mapping and navigation of internal body structures may be used, for example as described in more detail below and in commonly assigned US2022/0378498, the contents of which are hereby incorporated by reference in their entirety. Such systems may be impedance-based systems and/or magnetic-based localization systems. However, such systems may involve the use of additional circuitry such as coils or sensors, and/or processing circuitry and software. It would be desirable to provide systems and means for determining the relative orientation and/or positions of catheters without the need for such circuitry and software.

### Summary of the invention

Aspects of the invention are as set out in the independent claims and optional features are set out in the dependent claims. Aspects of the invention may be provided in conjunction with each other and features of one aspect may be applied to other aspects.

Fluoroscopy and/or ultrasound can be used to both assess occlusion of the vein and determine relative orientation of the balloon/catheter. While contrast injections may conventionally be used to assess occlusion, less or even no contrast may be used in embodiments of the disclosure.

In a first aspect there is provided an electroporation catheter. The electroporation catheter comprises a shaft, a plurality of splines forming a basket around a distal portion of the shaft, wherein each spline comprises at least one energizable electrode; and a balloon positioned within the basket formed by the plurality of splines, the balloon being selectively inflatable. The balloon comprises at least one radiopaque marking.

Advantageously, this method of marking the catheter is able to use the full diameter of the expanded balloon while providing pitch, roll, and yaw information relative to the patient's anatomy. Marking the balloon may also eliminate the need to use contrast during a procedure.

The radiopaque marking may comprise an elastic radiopaque ink and the radiopaque marking is configured to expand or contract as the balloon expands or contracts.

The radiopaque marking may be asymmetric. Asymmetric markers enable orientation determination.

The radiopaque marking may be applied to the inner diameter of the balloon. The radiopaque marking may be applied to the outer diameter of the balloon. Additionally or alternatively, the radiopaque marking is embedded into the material of the balloon.

The balloon may comprise at least two radiopaque markings.

The at least one radiopaque marking may be applied by pad printing and/or painting. Additionally, or alternatively, the at least one radiopaque marking may be applied by painting.

The at least one radiopaque marking may comprise a radiopaque ink applied during manufacturing. Additionally, or alternatively, the at least one radiopaque marking may comprise a sticker adhered to the balloon.

Each spline may extend between a proximal end that is coupled to the shaft and distal end that is coupled to the shaft. The shaft comprises an outer shaft element; and an inner shaft element extending through the outer shaft element. The distal end of each spline is operatively connected to a distal end of the inner shaft element. The proximal end of each spline is connected to a distal end of the outer shaft element. The inner shaft element is slidable relative to the outer shaft element to adjust an effective diameter of the basket formed by the plurality of splines. The electroporation catheter may further comprise a valve for selectively deflating and inflating the balloon to occupy the space within the plurality of splines.

In another aspect there is provided a balloon for an electroporation system, the balloon being selectively inflatable, the balloon comprising at least one radiopaque marking.

The radiopaque marking may comprise an elastic radiopaque ink and the radiopaque marking is configured to expand or contract as the balloon expands or contracts.

The radiopaque marking may be asymmetric.

The radiopaque marking may be applied to the inner diameter of the balloon and/or the outer diameter of the balloon. Additionally, or alternatively, the radiopaque marking may be embedded into the material of the balloon.

The balloon may comprise at least two radiopaque markings.

In another aspect there is provided an electroporation system. The electroporation system comprises a generator and a catheter (such as the electroporation catheter described above) coupled to the generator. The catheter comprises a handle, a shaft extending distally from the handle, a plurality of splines forming a basket around a distal portion of the shaft, wherein each spline comprises at least one energizable electrode, and a balloon positioned within the basket formed by the plurality of splines, the balloon comprising at least one radiopaque marking. The balloon may be the balloon of any of the aspects described above.

In another aspect there is provided a method of deploying an electroporation catheter assembly, such as the electroporation catheter assembly described above in another aspect. The method comprises delivering the electroporation catheter to a target site; deploying splines from the catheter to form a basket around a central shaft; inflating a balloon to occupy the space between the splines and conform to the shape of the basket; and locating the balloon using one or more radiopaque markers on the balloon.

### Drawings

Embodiments of the disclosure will now be described, by way of example only, with reference to the accompanying drawings, in which:
FIG. 1 is a schematic and block diagram view of a system for electroporation therapy.
FIG. 2 is a perspective view of an alternative embodiment of a catheter assembly.
FIG. 3 is a side schematic view of the catheter assembly shown in FIG. 2.
FIG. 4 is a side cross-sectional view of the catheter assembly shown in FIG. 2.
FIG. 5 is a schematic side cross-sectional view of the catheter assembly shown in FIG. 4.
FIG. 6 is another schematic side cross-sectional view of the catheter assembly shown in FIG. 4.
FIG. 7 is another schematic side cross-sectional view of the catheter assembly shown in FIG. 4.
FIG. 8 is a perspective view of an alternative embodiment of a catheter assembly.
FIGS. 9A-9C are perspective views of an example embodiment of a catheter assembly.
FIG. 10 is a process flow chart for a method of operating a catheter assembly, such as the catheter assembly of any of FIGS. 9A-9C.

### Detailed description

Systems and methods for electroporation catheters are described herein. An example electroporation catheter includes a shaft, and a plurality of splines forming a basket around a distal portion of the shaft, each spline extending between a proximal end that is coupled to the shaft and a distal end that is coupled to the shaft, wherein each spline of the plurality of splines comprises at least one energizable electrode. The electroporation catheter further includes a balloon positioned within the basket formed by the plurality of splines. The balloon comprises at least one radiopaque marking.

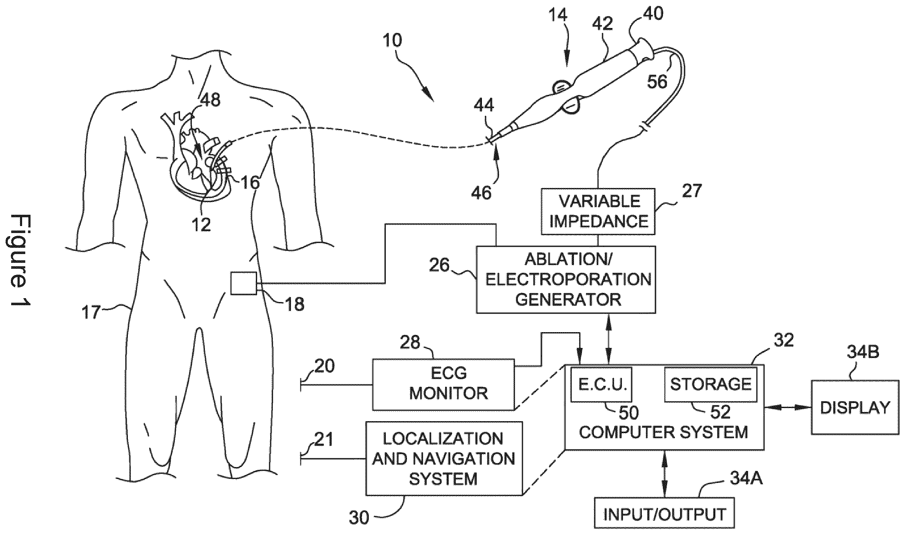

FIG. 1 is a block diagram view of an example system 10 for electroporation therapy. In general, system 10 includes a catheter electrode assembly 12 disposed at a distal end 48 of a catheter 14. As used herein, "proximal" refers to a direction toward the end of the catheter near the clinician and "distal" refers to a direction away from the clinician and (generally) inside the body of a patient. The electrode assembly includes one or more individual, electrically-isolated electrode elements. Each electrode element, also referred to herein as a catheter electrode, is individually wired such that it can be selectively paired or combined with any other electrode element to act as a bipolar or a multi-polar electrode.

System 10 may be used for irreversible electroporation (IRE) to destroy tissue. In particular, system 10 may be used for electroporation-induced primary apoptosis therapy, which refers to the effects of delivering electrical current in such a manner as to directly cause an irreversible loss of plasma membrane (cell wall) integrity leading to its breakdown and cell apoptosis. This mechanism of cell death may be viewed as an "outside-in" process, meaning that the disruption of the outside wall of the cell causes detrimental effects to the inside of the cell. Typically, for classical plasma membrane electroporation, electric current is delivered as a pulsed electric field in the form of short-duration pulses (e.g., having a 0.1 to 20 millisecond (ms) duration) between closely spaced electrodes capable of delivering an electric field strength of about 0.1 to 1.0 kilovolts/centimeter (kV/cm). System 10 may be used, for example, with a basket and/or balloon catheter assembly for high output (e.g., high voltage and/or high current) electroporation procedures. In some particular embodiments, system 10 is configured to deliver an electroporation pulse signal having a relatively high voltage and low pulse duration.

In one embodiment, all electrodes of the catheter deliver an electric current simultaneously. Alternatively, in other embodiments, stimulation is delivered between pairs of electrodes on the catheter. Delivering electric current simultaneously using a plurality of electrodes may facilitate creating a sufficiently deep lesion for electroporation. In some examples, to facilitate activating electrodes simultaneously, the electrodes may be switchable between being connected to a 3D mapping system, as will be described in more detail below, and being connected to EP amplifiers.

Irreversible electroporation using the catheters described herein may enable pulmonary vein isolation in as few as one shock per vein, which may produce much shorter procedure times compared to sequentially positioning a radiofrequency (RF) ablation tip around a vein.

It should be understood that while the energization strategies are described as involving DC pulses, embodiments may use variations and remain within the spirit and scope of the disclosure. For example, exponentially-decaying pulses, exponentially-increasing pulses, and combinations may be used. Further, in some embodiments, AC pulses may be used.

Further, it should be understood that the mechanism of cell destruction in electroporation is not primarily due to heating effects, but rather to cell membrane disruption through application of a high-voltage electric field. Thus, electroporation may avoid some possible thermal effects that may occur when using radio frequency (RF) energy. This "cold therapy" thus has desirable characteristics.

With this background, and now referring again to FIG. 1, system 10 includes a catheter electrode assembly 12 including at least one catheter electrode. Electrode assembly 12 is incorporated as part of a medical device such as a catheter 14 for electroporation therapy of tissue 16 in a body 17 of a patient. In the illustrative embodiment, tissue 16 includes heart or cardiac tissue. It should be understood, however, that embodiments may be used to conduct electroporation therapy with respect to a variety of other body tissues.

FIG. 1 further shows a plurality of return electrodes designated 18, 20, and 21, which are diagrammatic of the body connections that may be used by the various sub-systems included in overall system 10, such as an electroporation generator 26, an electrophysiology (EP) monitor such as an ECG monitor 28, and an optional localization and navigation system 30 for visualization, mapping, and navigation of internal body structures. In the illustrated embodiment, return electrodes 18, 20, and 21 are patch electrodes. It should be understood that the illustration of a single patch electrode is diagrammatic only (for clarity) and that such sub-systems to which these patch electrodes are connected may, and typically will, include more than one patch (body surface) electrode, and may include split patch electrodes (as described herein). In other embodiments, return electrodes 18, 20, and 21 may be any other type of electrode suitable for use as a return electrode including, for example, one or more catheter electrodes. Return electrodes that are catheter electrodes may be part of electrode assembly 12 or part of a separate catheter or device (not shown). System 10 may further include a main computer system 32 (including an electronic control unit 50 and data storage-memory 52), which may be integrated with optional localization and navigation system 30 in certain embodiments. System 32 may further include conventional interface components, such as various user input/output mechanisms 34A and a display 34B, among other components.

Electroporation generator 26 is configured to energize the electrode element(s) in accordance with an electroporation energization strategy, which may be predetermined or may be user-selectable. For electroporation-induced primary apoptosis therapy, generator 26 may be configured to produce an electric current that is delivered via electrode assembly 12 as a pulsed electric field in the form of short-duration DC pulses (e.g., a nanosecond to several milliseconds duration, a 0.1 to 20 ms duration, or any duration suitable for electroporation) between closely spaced electrodes capable of delivering an electric field strength (i.e., at the tissue site) of about 0.1 to 1.0 kV/cm. The amplitude and pulse duration needed for irreversible electroporation are inversely related. As pulse durations are decreased, the amplitude must be increased to achieve electroporation.

Electroporation generator 26, sometimes also referred to herein as a DC energy source, is a monophasic electroporation generator 26 configured to generate a series of DC energy pulses that all produce current in the same direction. In other embodiments, electroporation generator is biphasic or polyphasic electroporation generator configured to produce DC energy pulses that do not all produce current in the same direction. In some embodiments, electroporation generator 26 is configured to output energy in DC pulses at selectable energy levels, such as fifty joules, one hundred joules, two hundred joules, and the like. Other embodiments may have more or fewer energy settings and the values of the available setting may be the same or different. For successful electroporation, some embodiments utilize the two hundred joule output level. For example, electroporation generator 26 may output a DC pulse having a peak magnitude from about 300 Volts (V) to about 3,200 V at the two hundred joule output level. In some embodiments, the peak magnitude may be even larger (e.g., on the order of 10,000 V). Other embodiments may output any other suitable positive or negative voltage. For example, in some embodiments, the systems and methods described herein may include pulses with amplitudes from about 500 V to about 4,000 V, with pulse widths from about 200 nanoseconds to about 20 microseconds.

In some embodiments, a variable impedance 27 allows the impedance of system 10 to be varied to limit arcing. Moreover, variable impedance 27 may be used to change one or more characteristics, such as amplitude, duration, pulse shape, and the like, of an output of electroporation generator 26. Although illustrated as a separate component, variable impedance 27 may be incorporated in catheter 14 or generator 26.

With continued reference to FIG. 1, as noted above, catheter 14 may include functionality for electroporation and in certain embodiments also other types of ablation (e.g., RF ablation). It should be understood, however, that in those embodiments, variations are possible as to the type of ablation energy provided (e.g., cryoablation, ultrasound, etc.).

In the illustrative embodiment, catheter 14 includes a cable connector or interface 40, a handle 42, and a shaft 44 having a proximal end 46 and a distal 48 end. Catheter 14 may also include other conventional components not illustrated herein such as a temperature sensor, additional electrodes, and corresponding conductors or leads. Connector 40 provides mechanical and electrical connection(s) for cable 56 extending from generator 26. Connector 40 may include conventional components known in the art and as shown is disposed at the proximal end of catheter 14.

Handle 42 provides a location for the clinician to hold catheter 14 and may further provide means for steering or the guiding shaft 44 within body 17. For example, handle 42 may include means to change the length of a guidewire extending through catheter 14 to distal end 48 of shaft 44 or means to steer shaft 44. Moreover, in some embodiments, handle 42 may be configured to vary the shape, size, and/or orientation of a portion of the catheter, and it will be understood that the construction of handle 42 may vary. In an alternate embodiment, catheter 14 may be robotically driven or controlled. Accordingly, rather than a clinician manipulating a handle to advance/retract and/or steer or guide catheter 14 (and shaft 44 thereof in particular), a robot is used to manipulate catheter 14. Shaft 44 is an elongated, tubular, flexible member configured for movement within body 17. Shaft 44 is configured to support electrode assembly 12 as well as contain associated conductors, and possibly additional electronics used for signal processing or conditioning. Shaft 44 may also permit transport, delivery and/or removal of fluids (including irrigation fluids and bodily fluids), medicines, and/or surgical tools or instruments. Shaft 44 may be made from conventional materials such as polyurethane and defines one or more lumens configured to house and/or transport electrical conductors, fluids or surgical tools, as described herein. Shaft 44 may be introduced into a blood vessel or other structure within body 17 through a conventional introducer. Shaft 44 may then be advanced/retracted and/or steered or guided through body 17 to a desired location such as the site of tissue 16, including through the use of guidewires or other means known in the art.

In some embodiments, catheter 14 includes a basket catheter assembly having catheter electrodes (not shown in FIG. 1) distributed at the distal end of shaft 44 in a basket structure. Further, as described herein, an inflatable balloon may be contained within the basket structure.

Pulsed field ablation (PFA) has been shown to be an effective form of ablation for treatment of cardiac arrhythmias, particularly for instantaneous pulmonary vein isolation (PVI). PFA includes delivering high voltage pulses from electrodes disposed on a catheter (e.g., including the basket and/or balloon catheters described herein). In PFA, for example, voltage amplitudes may range from about 300 V to at least 3,200 V (or even as large as on the order as 10,000 V), and pulse widths may from hundreds of nanoseconds to tens of milliseconds.

These electric fields may be applied between adjacent electrodes (in a bipolar approach) or between a one or more electrodes and a return patch (in a monopolar approach). There are advantages and disadvantages to each of these approaches (e.g., when using the basket and/or balloon catheters described herein).

For example, regarding lesion contiguity, the monopolar approach has the potential to leave gaps in lesion coverage (referred to as dead zones) between electrodes where the field strength is low or zero, whereas the field strength in the bipolar approach generally prevents dead zones between electrodes.

For lesion size and proximity, the monopolar approach has a wider range of effect, and can potentially create deeper lesions with the same applied voltage. Further, the monopolar approach may be able to create lesions from a distance (e.g., generally proximate, but not necessarily contacting tissue). The bipolar approach may create smaller lesions, requiring closer proximity or contact with tissue to create transmural lesions. However, the monopolar approach may create larger lesions than are necessary, while the lesions generated using the bipolar approach may be more localized.

Due to a wider range of effect, the monopolar approach may cause unwanted skeletal muscle and/or nerve activation. In contrast, the bipolar approach has a constrained range of effect proportional to electrode spacing on the spine, and is less likely to depolarize cardiac myocytes or nerve fibers.

For the monopolar approach, only a single potential is applied in catheter wires and electrodes. Further, because all the electrodes are at the same polarity, the configuration is not susceptible to arcing (e.g., when using the basket and/or balloon catheters described herein). In contrast, for the bipolar approach, the internal architecture of the catheter must be constructed to prevent arcing, as different electrodes are at different potentials.

To monitor operation of system 10, one or more impedances between catheter electrodes and/or return electrodes 18, 20, and 21 may be measured. For example, for system 10, impedances may be measured as described in U.S. Patent Application Publication No. 2019/0117113, filed on Oct. 23, 2018, U.S. Patent Application Publication No. 2019/0183378, filed on Dec. 19, 2018, and U.S. Patent Application No. 63/027,660, filed on May 20, 2020, all of which are incorporated by reference herein in their entirety.

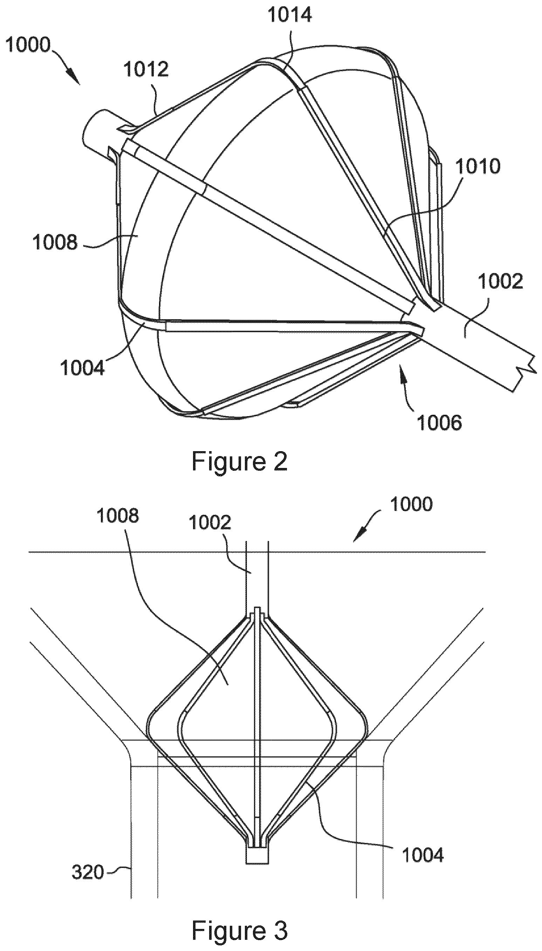

FIG. 2 is a perspective view of an example embodiment of a catheter assembly 1000, and FIG. 3 is a side schematic view of catheter assembly 1000. Catheter assembly 1000 includes a shaft 1002 and a plurality of splines 1004 surrounding a distal portion 1006 of shaft 1002. Catheter assembly 1000 includes a balloon 1008 enclosed by splines 1004. Balloon 1008 may be selectively inflated to occupy the space between splines 1004. Notably, balloon 1008 functions as an insulator, and generally reduces energy losses relative to catheter assembly 1000, which may result in increased lesion size.

Splines 1004 may all be electrically connected to one another as a single electrode, or may each be individual electrodes. When splines 1004 are individual electrodes, each spline 1004 may be selectively energizable to form different energization sequences and/or patterns. In general, each spline 1004 has the same polarity, to avoid arcing issues.

Each spline 1004 includes a proximal end 1010 coupled to shaft 1002 and a distal end 1012 coupled to shaft 1002. From proximal end 1010, spline 1004 extends radially outward to an inflection point 1014, and then extends radially inward to distal end 1012. FIG. 3 shows catheter assembly 1000 positioned within pulmonary vein 320.

A body of each spline 1004 is made of an elastic material (e.g., Nitinol), stainless steel, and/or other superalloys. Splines 1004 may be formed, for example, by laser cutting a tubing (e.g., made of Nitinol, stainless steel, and/or other superalloys) into multiple strips, and heat treating the strips to form a shape that conforms with balloon 1008. The body of each spline 1004 and functions as a relatively large electrode. Further, the entirety of each spline 1004 may serve as an electrode, or portions of each spline 1004 may be covered with an insulating material (e.g., a polyethylene terephthalate (PET) heat shrink material or polyether block amide (PEBA) material) such that only non-insulation portions of each spline 1004 serve as electrodes. Further, in some implementations, multiple independently energizable electrodes are attached to each spline 1004.

In the example shown, alternating splines 1004 alternate polarities. That is, each positive spline 1004 is positioned between two negative splines 1004 and vice-versa. Alternatively, any suitable polarization scheme may be used.

During delivery, splines 1004 and balloon 1008 may be collapsed. To perform ablation, splines 1004 are deployed with inflection points 1014 extending radially outward, and balloon 1008 is selectively inflated to occupy the space between splines 1004.

In some embodiments, the shape of balloon 1008 may be selectively changed to improve ablation. For example, splines 1004 may be axially compressed from a first configuration to a second configuration, such that the effective diameter of splines 1004 is greater in the second configuration than the first configuration.

To facilitate selectively transitioning balloon catheter assembly 1000 between first and second configurations, an inner shaft member may be slidably positioned within shaft 1002, with a distal end of inner shaft member coupled to a distal end of balloon 1008 and splines 1004. When the inner shaft member is pulled proximally relative to shaft 1002, the distal end of balloon 1008 and splines 1004 are also pulled proximally relative to a proximal end of balloon 1008 and splines 1004, thereby compressing balloon 1008 and splines 1004 axially. The position of the inner shaft member relative to shaft 1002 may be held in place using a suitable locking mechanism (e.g., a Tuohy Borst compression valve), as described in detail herein.

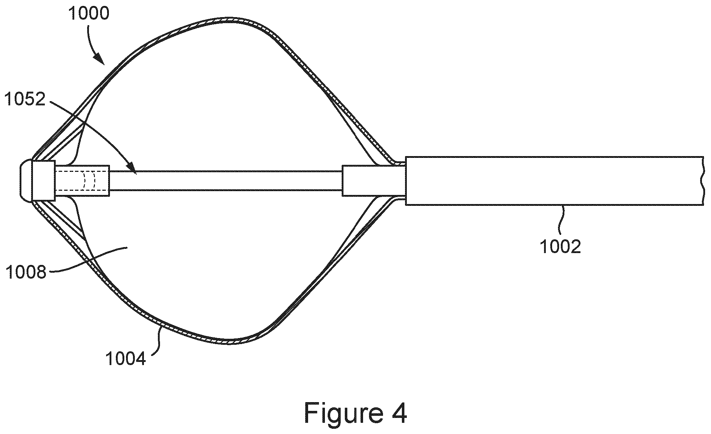

FIG. 4 is a side cross-sectional view of catheter assembly 1000. In this embodiment, central lumen 1052 facilitates delivering a fluid to the interior of balloon 1008 to selectively inflate balloon 1008. The fluid may include saline or a mixture of saline and contrast agent. In some embodiments, shaft 1002 may include irrigation holes (not shown) that provide fluid communication between central lumen 1052 and the interior of balloon 1008. Further, in some embodiments, central lumen 1052 enables infusing contrast agent distal of catheter assembly 1000, which may assist a user in assessing blood flow through pulmonary vein 320 and assessing how much catheter assembly 1000 occludes pulmonary vein 320.

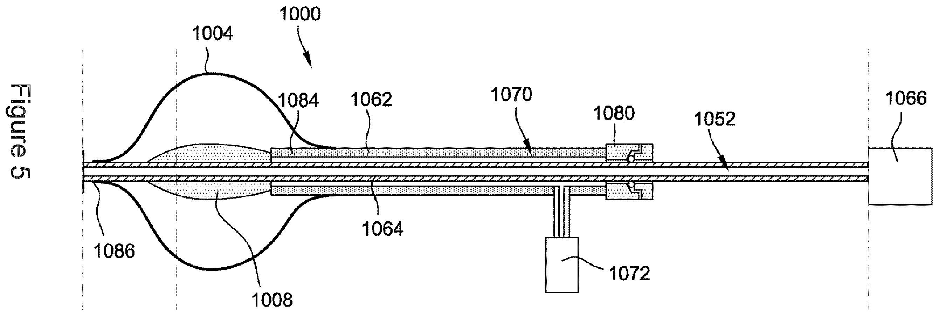

FIGS. 5-7 are schematic side cross-sectional views of catheter assembly 1000 illustrating the transitioning of catheter assembly 1000 between different states. As shown in FIG. 5, in this embodiment, shaft 1002 includes an outer shaft element 1062 and an inner shaft element 1064, both of which are tubular components. Inner shaft element 1064 is axially slidable within outer shaft element 1062, and defines central lumen 1052 therethrough. Outer shaft element 1062 may have a French size of, for example, 11.5 French. Alternatively, outer shaft element 1062 may have any suitable dimensions.

A valve 1066 at a distal end of inner shaft element 1064 controls access to central lumen 1052. For example, as noted above, contrast agent may be flushed through central lumen 1052 into the patient to confirm pulmonary vein occlusion. As another example, a mapping catheter (e.g., a 3 French mapping catheter) may extend into the patient through central lumen 1052. As yet another example, a guidewire may extend through central lumen 1052. Those of skill in the art will appreciate that occlusion may be monitored using any suitable technique. For example, pressure monitoring may be used to assess occlusion of the vein, contrast injection may be used to assess occlusion of the vein using fluoroscopy, and/or ultrasound (e.g., Doppler) may be used to assess occlusion.

As shown in FIG. 5, a channel 1070 is defined between outer shaft element 1062 and inner shaft element 1064 in this embodiment. Channel is in fluid communication with an interior of balloon 1008. Further, a stopcock valve 1072 (e.g., a three-way stopcock valve) enables inflating and deflating balloon as desired, by controlling fluid flow to the interior of balloon 1008.

In this example embodiment, catheter assembly 1000 further includes a compression valve 1080 that facilitates securing a position of outer shaft element 1062 relative to inner shaft element 1064. Specifically, with compression valve 1080 open, inner shaft element 1064 is slidable relative to outer shaft element 1062. Once inner shaft element 1064 is located at a desired position, compression valve 1080 may be closed to prevent inner shaft element 1064 from sliding relative to outer shaft element 1026. Compression valve 1080 also seals off a proximal end of channel 1070.

As shown in FIG. 5, each spline 1004 extends between a distal end 1084 of outer shaft element 1026 and a distal end 1086 of inner shaft element 1064. Accordingly, by sliding inner shaft element distal end 1086 relative to outer shaft element distal end 1084, the shape of splines 1004 is adjustable.

FIG. 5 shows splines 1004 in a neutral position, with balloon 1008 deflated. Specifically, in this embodiment, splines 1004 are made of a shape memory material (e.g., Nitinol) such that splines 1004 assume the shape shown in FIG. 5 when not subjected to any external forces or biases.

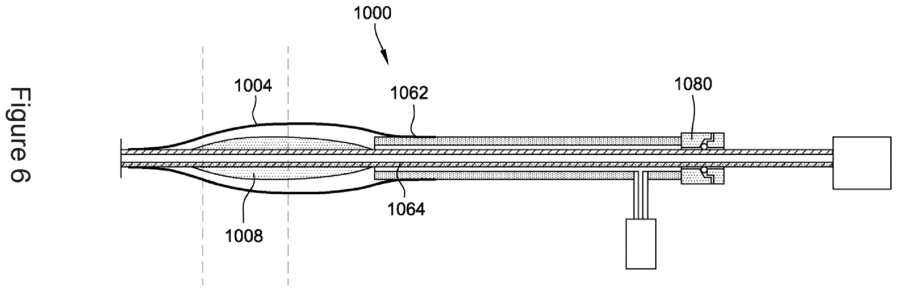

To compress catheter assembly 1000 (e.g., for delivery of catheter assembly 1000), inner shaft element 1064 is slid distally relative to outer shaft element 1062. This causes splines 1004 to collapse inward towards inner shaft element, transitioning catheter assembly 1000 to a collapsed position, as shown in FIG. 6. In FIG. 6, balloon 1008 is deflated. Catheter assembly 1000 may be locked in the collapsed position using compression valve 1080.

In some embodiments, one or more lumens (lumen) are defined within outer shaft element 1062 (e.g., between an inner and outer surface of outer shaft element 1062). The lumens may be used to route electrical wires (i.e., for supplying power to splines 1004) and/or activation wires (i.e., for controlling an orientation of catheter assembly 1000) through catheter assembly 1000. For example, in one embodiment, positive electrical wires that supply energy to positive splines 1004 are all routed through a first lumen, and negative electrical wires that supply energy to negative splines are all routed through a second, separate lumen. This effectively isolates the positive electrical wires from the negative electrical wires.

Each electrical wire may be coupled to an associated spline 1004, for example, through a weld, with the weld and proximal ends of the splines arranged on a proximal coupler component (not shown). Further, in some embodiments, a strain relief component (not shown) is coupled to an exterior of the inner shaft element 1064 distal of the outer shaft element 1062, to prevent inner shaft element 1064 from excess bending.

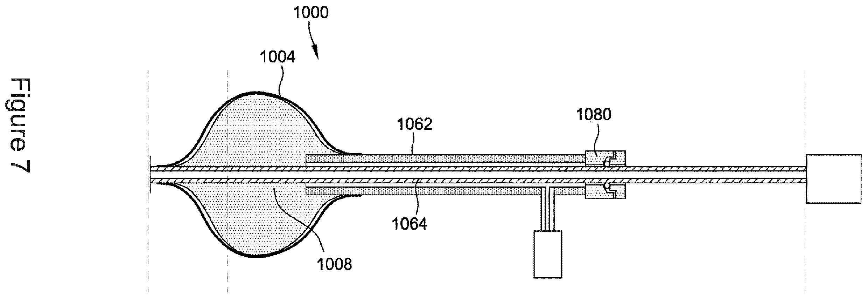

Referring now to FIG. 7, during therapy, balloon 1008 is inflated to fill the space between splines 1004. Specifically, as shown in FIG. 7, with balloon 1008 inflated, balloon 1008 generally conforms to the shape of the basket formed by splines 1004.

The basket formed by splines 1004 may also be compressed to increase an outer diameter of catheter assembly 1000. When inner shaft element 1064 is slid proximally relative to outer shaft element 1062, this causes splines 1004 to flex outward, transitioning catheter assembly 1000 to a compressed position. Catheter assembly 1000 may be locked in the compressed position using compression valve 1080.

In the compressed position, balloon 1008 may be inflated to fill the space between splines 1004. With balloon 1008 inflated, balloon 1008 generally conforms to the shape of the basket formed by splines 1004 in the compressed position.

Catheter assembly 1000 provides several advantages. For example, the combination of balloon 1008 and splines 1004 facilitates straightforward delivery and deployment of catheter assembly 1000. Further, balloon 1008 drives more energy into ablated tissue, and stabilizes splines 1004 to prevent lateral movement. In addition, using splines 1004 as electrodes instead of individual smaller electrodes facilitates reducing the cost and increasing the reliability of catheter assembly 1000. Furthermore, the balloon may act as an insulator for the blood inside the patient, reducing thermal effects such as bubble formation. The balloon may also help stabilize the basket inside the patient, for example in the pulmonary vein, where the device's round shape allows a physician to place the catheter at the ablation target site, apply energy, and rotate the device slightly to achieve a spline offset for a second energy application.

Splines 1004 may all have the same length, or at least some of splines 1004 may have different lengths. Further, insulating material (e.g., a PET or PEBA material) on each spline 1004 may have the same length, or at least some splines 1004 may have insulating material with different lengths. In addition, in some embodiments, catheter assembly 1000 includes a distal electrode (not shown) positioned distal of splines 1004. The distal electrode may be used to perform point ablation (e.g., by creating a bipole between the distal electrode and one of splines 1004), and/or may be used for visualization/mapping purposes (e.g., using the distal electrode in combination with an electrode on shaft 1002).

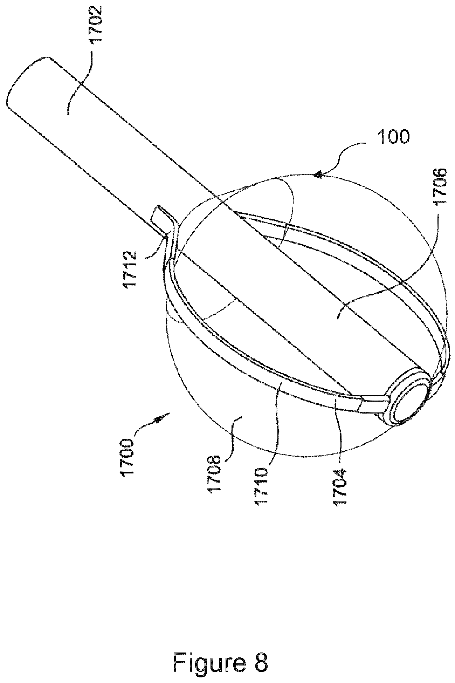

FIG. 8 is a perspective view of an alternative embodiment of a catheter assembly 1700. Catheter assembly 1700 includes a shaft 1702 and a plurality of splines 1704 surrounding a distal portion 1706 of shaft 1702. Catheter assembly 1700 also includes a balloon 1708 enclosed by splines 1704. Balloon 1708 may be selectively inflated to fill the space between splines 1704. Notably, balloon 1708 functions as an insulator, and generally reduces energy losses, which may result in increased lesion size.

Relative to catheter assembly 1000, catheter assembly 1700 may be smaller, with a more simplified design, which may result in reduced manufacturing costs. For example, in some embodiments, catheter assembly 1000 may have a diameter in a range of about 28-35 mm with the splines expanded, in order to facilitate performing PVI. In contrast, with splines 1704 expanded, catheter assembly 1700 may have a diameter in a range of about 8-10 mm (e.g., a diameter of 9 mm). Further, when collapsed, catheter assembly 1700 may be deliverable using a 7.5 French shaft.

The smaller diameter allows catheter assembly 1700 to generate smaller, more focused lesions. For example, catheter assembly 1700 may be used to treat gaps that remain after another catheter assembly has been used to perform PVI. Accordingly, catheter assembly 1700 may be used to supplement procedures performed using other catheter assemblies. As another example, catheter assembly 1700 may be used to treat other targets, such as the posterior wall.

From simulations, it has been demonstrated that catheter assembly 1700 generates lesions substantially consistently, regardless of the orientation of catheter assembly 1700 to target tissue. For example, in simulations, lesions having a depth of at least 4 mm were achieved with shaft 1702 at angles of approximately 10°, 45°, and 90° relative to a surface of the target tissue.

In the embodiment shown, each spline 1704 includes an exposed portion 1710 that functions as an electrode, and an insulated portion 1712 proximal of exposed portion 1710. In some embodiments, another insulated portion 1712 is included distal of exposed portion 1710. Alternatively, this distal insulated portion 1712 may be omitted. Although splines 1704 are shows as having a constant width, those of skill in the art will appreciate that other spline shapes may be used (e.g., tapered shapes, split shapes, etc.).

To perform ablation, a voltage (e.g., 1500 V) is applied to catheter assembly 1700. Specifically, the voltage may be applied between splines 1704 in accordance with a bipolar approach. Alternatively, the voltage may be applied between one or more splines 1704 and a separate electrode (e.g., a body patch electrode) under a monopolar approach. Further, voltages may be applied to splines 1704 concurrently, or sequentially (e.g., via multiplexing). While catheter assembly 1700 includes two splines 1704, those of skill in the art will appreciate that more splines may be included.

As noted above, localization and navigation systems may optionally be provided for visualization, mapping and navigation of internal body structures. Localization and navigation system 30 may include conventional apparatus known generally in the art (e.g., an EnSite Precision^{™} System, commercially available from Abbott Laboratories. and as generally shown with reference to commonly assigned U.S. Pat. No. 7,263,397 titled "Method and Apparatus for Catheter Navigation and Location and Mapping in the Heart," the entire disclosure of which is incorporated herein by reference). It should be understood, however, that this system is an example only, and is not limiting in nature. Other technologies for locating/navigating a catheter in space (and for visualization) are known, including for example, the CARTO navigation and location system of Biosense Webster, Inc., the Rhythmia^{®} system of Boston Scientific Scimed, Inc., the KODEX^{®} system of Koninklijke Philips N.V., the AURORA^{®} system of Northern Digital Inc.,, or a magnetic location system such as the gMPS system from Mediguide Ltd. In this regard, some of the localization, navigation and/or visualization systems would involve a sensor be provided for producing signals indicative of catheter location information, and may include, for example one or more electrodes in the case of an impedance-based localization system, or alternatively, one or more coils (i.e., wire windings) configured to detect one or more characteristics of a magnetic field, for example in the case of a magnetic-field based localization system. As yet another example, system 10 may utilize a combination electric field-based and magnetic field-based system as generally shown with reference to U.S. Pat. No. 7,536,218 entitled "Hybrid Magnetic-Based and Impedance Based Position Sensing," the disclosure of which is incorporated herein by reference in its entirety.

However, as noted above, in some embodiments localization and navigation system 30 is optional and may not be present. In such examples the balloon 1008, 1708 may comprise at least one radiopaque marking. Advantageously, the use of a radiopaque marking on the balloon means that the relative position and orientation of the balloon 1008, 1708 may be determined without the use of localization and navigation system 30. For example, the relative orientation and position of the balloon 1008, 1708 may be determined using fluoroscopy or ultrasound.

Providing a radiopaque marking on the balloon 1008, 1708 itself means that the full size of the balloon 1008, 1708 may be used to determine the relative position and orientation of the balloon 1008, 1708 and therefore the catheter assembly 1000. Using the full size of the balloon 1008, 1708 makes the radiopaque markings easier to see (e.g., under fluoroscopy).

The radiopaque marking may be elastic, meaning that the radiopaque marking can expand and contract as the balloon 1008, 1708 is inflated and deflated.

The radiopaque marking may be asymmetric, meaning that the relative orientation of the balloon 1008, 1708 to which the marking is applied can be readily determined. For example, pitch, roll, and yaw information relative to the patient's anatomy may be determined.

The radiopaque marking may be a radiopaque ink. For example, the radiopaque marking may be an elastic radiopaque ink. For example, the radiopaque marking may be manufactured by Creative Materials, Inc (12 Willow Road, Ayer, MA 01432, USA). For example, the radiopaque marking may be 113-49 pad-printable radiopaque ink, or 125-09MDB medium durometer blue radiopaque ink. In other examples the radiopaque marking may be a radiopaque sticker that adheres to the balloon 1008 (for example to the inner face or inner diameter of the balloon 1008, or to the outer face or outer diameter of the balloon 1008). For example, the radiopaque sticker may comprise radiopaque or metallic ink that is applied.

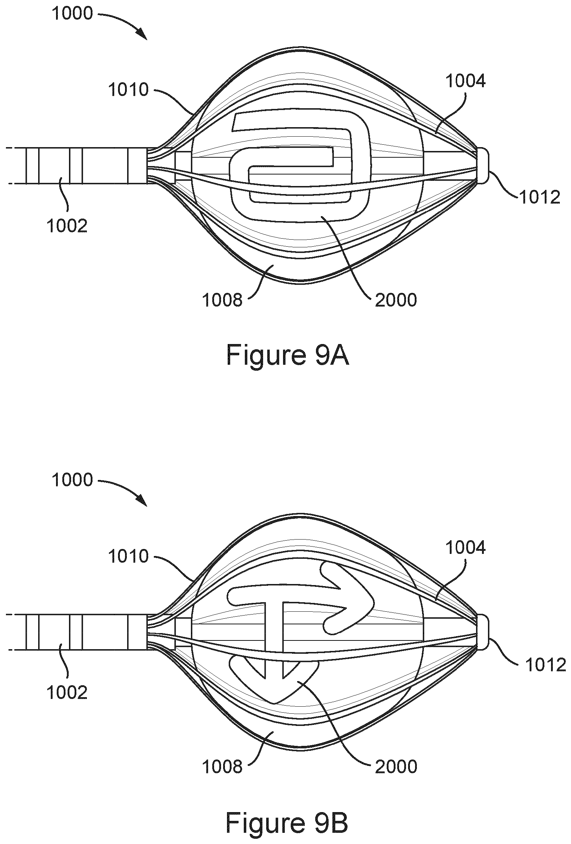

FIGS. 9A-9C show perspective views of example catheter assemblies 1000 comprising balloons 1008 with radiopaque markings 2000. The catheter assemblies 1000 shown in FIGS. 9A to 9C are very similar to the catheter assemblies 1000 of FIGS. 2 to 7 as described above, in that the balloon 1008 is surrounded by a basket formed by splines 1004, however it will be understood that other catheter assemblies may be used, such as the catheter assembly 1700 of FIG. 8. In the example embodiments shown in FIGS. 9A-9C, each spline 1004 extends between a proximal end 1010 that is coupled to the shaft 1002 and distal end 1012 that is coupled to the shaft 1002. The shaft 1002 comprises an outer shaft element 1062 and an inner shaft element 1064 extending through the outer shaft element 1062 (for example as shown in FIG. 5). The distal end 1012 of each spline 1004 is operatively connected to a distal end 1086 of the inner shaft element 1064. The proximal end 1010 of each spline 1004 is connected to a distal end 1084 of the outer shaft element 1062, and wherein the inner shaft element 1064 is slidable relative to the outer shaft element 1062 to adjust an effective diameter of the basket formed by the plurality of splines 1004. Also as shown in FIG. 5, the catheter assembly 1000 also comprises a channel 1070 that is defined between outer shaft element 1062 and inner shaft element 1064. Channel 1070 is in fluid communication with an interior of balloon 1008. Further, a stopcock valve 1072 (e.g., a three-way stopcock valve) enables inflating and deflating balloon as desired, by controlling fluid flow to the interior of balloon 1008.

As described above with reference to FIG. 7, during therapy, balloon 1008 of FIGS. 9A to 9C is inflated to fill the space between splines 1004. Specifically, as shown in FIGS. 9A to 9C, with balloon 1008 inflated, balloon 1008 generally conforms to the shape of the basket formed by splines 1004. This means that the radiopaque markings 2000 are large and easily seen by an operator of the catheter assembly 1000 e.g., via fluoroscopy or ultrasound imaging.

FIG. 9A shows a first example of a radiopaque marking 2000 on a balloon 1008 of a catheter assembly 1000. The radiopaque marking 2000 in the example of FIG. 9A is in the form of an "a". In this example, the radiopaque marking 2000 is an elastic ink applied by painting to the outer face or outer diameter of the balloon 1008, however it will be understood that in other examples the radiopaque marking 2000 may be applied to the inner face or inner diameter of the balloon, and radiopaque marking 2000 may be applied via other techniques such as pad printing. In yet further examples, the radiopaque marking 2000 may be embedded into the material of the balloon 1008 itself. For example, the radiopaque marking 2000 may be applied during manufacture (e.g., a radiopaque substance such as a metallic ink may be embedded into the balloon 1008 during manufacture).

FIG. 9B shows another example of a radiopaque marking 2000 on a balloon 1008 of a catheter assembly 1000. The radiopaque marking 2000 in the example of FIG. 9A is in the form of a pair of arrows 90 degrees apart from each other. Again, in this example, the radiopaque marking 2000 is an elastic ink applied by painting to the outer face or outer diameter of the balloon 1008, however it will be understood that in other examples the radiopaque marking 2000 may be applied to the inner face or inner diameter of the balloon, and radiopaque marking 2000 may be applied via other techniques such as pad printing. In yet further examples, the radiopaque marking 2000 may be embedded into the material of the balloon 1008 itself. For example, the radiopaque marking 2000 may be applied during manufacture (e.g., a radiopaque substance such as a metallic ink may be embedded into the balloon 1008 during manufacture).

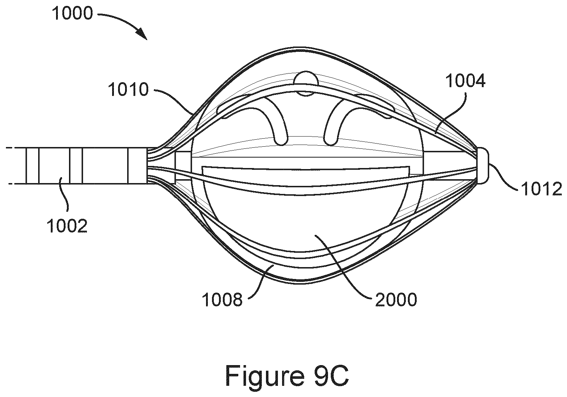

FIG. 9C shows another example of a radiopaque marking 2000 on a balloon 1008 of a catheter assembly 1000. The radiopaque marking 2000 in the example of FIG. 9A is in the form of an attitude indicator. In this example the attitude indicator includes an artificial horizon with solid shading below the horizon and directional markings above the horizon. Again, in this example, the radiopaque marking 2000 is an elastic ink applied by painting to the outer face or outer diameter of the balloon 1008, however it will be understood that in other examples the radiopaque marking 2000 may be applied to the inner face or inner diameter of the balloon, and radiopaque marking 2000 may be applied via other techniques such as pad printing. In yet further examples, the radiopaque marking 2000 may be embedded into the material of the balloon 1008 itself. For example, the radiopaque marking 2000 may be applied during manufacture (e.g., a radiopaque substance such as a metallic ink may be embedded into the balloon 1008 during manufacture).

Although only one radiopaque marking 2000 is shown in the examples shown in FIGS. 9A to 9C, it will be appreciated that in other examples there may be more than one radiopaque marking 2000. For example, there may be a plurality of radiopaque markings 2000 applied to the balloon 1008. The plurality of radiopaque markings 2000 may be of the same shape or of different shapes.

In some examples radiopaque markings 2000 may be applied to different portions of the balloon 1008 to improve the intelligibility of the orientation of the balloon 1008. For example, radiopaque markings may be applied to the balloon 1008 offset from each around the circumference of the balloon 1008. For example, radiopaque markings 2000 may be applied to the balloon 1008 such that they are offset from each other by a selected angle, for example, at least 90 degrees around the circumference of the balloon 1008. The radiopaque markings 2000 offset from each other may be of the same shape or have different shapes to further improve intelligibility of the orientation of the balloon 1008.

Additionally, or alternatively, radiopaque markings may be applied to opposing sides of the balloon 1008. In some examples, radiopaque markings 2000 may be applied in opposing pairs. Each opposing pair may comprise radiopaque markings 2000 of a particular shape. For example, a first pair of radiopaque markings 2000 may have the marking shown in FIG. 9A, whereas a second pair of radiopaque markings may have the marking shown in FIG. 9B or 9C.

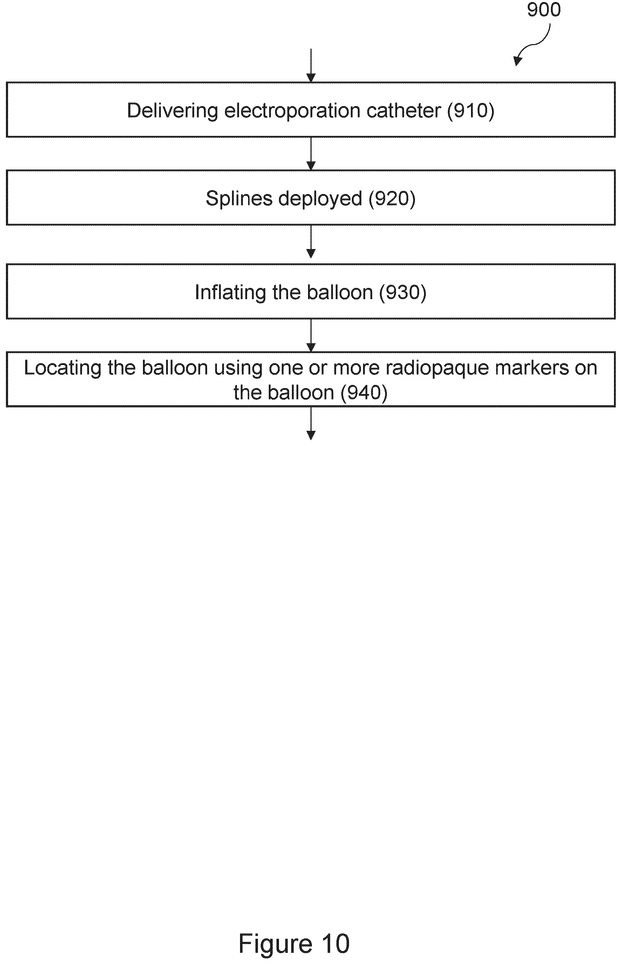

A method of deploying a catheter assembly, such as the catheter assembly 1000 described above, will now be discussed with reference to FIG. 10. For delivery of catheter assembly 1000, the catheter assembly 1000 is first compressed by sliding inner shaft element 1064 distally relative to outer shaft element 1062. This causes splines 1004 to collapse inward towards inner shaft element, transitioning catheter assembly 1000 to a collapsed position (as shown in FIG. 6), when the balloon 1008 is deflated. Catheter assembly 1000 may be locked in the collapsed position using compression valve 1080.

The electroporation catheter assembly 1000 is then delivered 910 to the target site. This may, for example, within the pulmonary vein, as shown in FIG. 3 discussed above. However, it will be understood that the target site may not be in or on the human body, for example the target site may be in or on a cadaver.

Once the electroporation catheter assembly 1000 is at the target site, the splines 1004 are deployed 920 with inflection points 1014 extending radially outward, and balloon 1008 is selectively inflated 930 to occupy the space between splines 1004. As shown in FIG. 7, as discussed above, with balloon 1008 inflated, balloon 1008 generally conforms to the shape of the basket formed by splines 1004. It will be understood that the shape of balloon 1008 may be selectively changed to improve ablation. For example, splines 1004 may be axially compressed from a first configuration to a second configuration, such that the effective diameter of splines 1004 is greater in the second configuration than the first configuration. For example, when inner shaft element 1064 is slid proximally relative to outer shaft element 1062, this causes splines 1004 to flex outward, transitioning catheter assembly 1000 to a compressed position. Catheter assembly 1000 may be locked in the compressed position using compression valve 1080.

Once the splines 1004 are deployed and the balloon inflated, the user may wish to locate 940 the balloon and thereby the catheter assembly 1000 and splines 1004 relative to the target site. This may be performed using imaging such as fluoroscopy or ultrasound imaging. Using fluoroscopy or ultrasound imaging, the radiopaque marker(s) 2000 on the balloon 1008 may be readily identified as regions of much greater contrast, and the relative position and orientation (e.g., pitch, yaw and roll) of the catheter assembly 1000 and therefore the splines 1004 determined. Determining the relative position and orientation of the catheter assembly 1000 and splines 1004 means that the catheter assembly 1000 and splines 1004 can be positioned accurately at the target site for treatment without causing any unnecessary damage to surrounding tissue.

As discussed above, in some embodiments, contrast agent may be infused distal of catheter assembly 1000, which may assist a user in assessing blood flow through pulmonary vein 320 and assessing how much catheter assembly 1000 occludes pulmonary vein 320. Because the radiopaque markers 2000 enable the location and relative position and orientation of the catheter assembly 1000 (and specifically the balloon 1008 and splines 1004) relative to the tissue/target site be determined, the use of the radiopaque markers 2000 may reduce or eliminate the need for any contrast to be used.

Once the catheter assembly 1000 is determined to be at the target site, if it is determined that treatment is required, an electroporation generator (such as the electroporation generator 26 described above with reference to FIG. 1) may be operated to energize the electrode elements in accordance with an electroporation energization strategy, which may be predetermined or user-selectable. For example, for electroporation-induced primary apoptosis therapy, generator 26 may be configured to produce an electric current that is delivered via electrode assembly 12 as a pulsed electric field in the form of short-duration DC pulses (e.g., a nanosecond to several milliseconds duration, a 0.1 to 20 ms duration, or any duration suitable for electroporation) between closely spaced electrodes capable of delivering an electric field strength (i.e., at the tissue site) of about 0.1 to 1.0 kV/cm.

It will be understood that even if a localization and navigation system such as localization and navigation system 30 described above with reference to FIG. 1 is not used, the ablation/electroporation generator 26 can still be controlled independently to selectively energize each of the splines 1004 (or more specifically the electrodes on each of the splines 1004).

Although certain embodiments of this disclosure have been described above with a certain degree of particularity, those skilled in the art could make numerous alterations to the disclosed embodiments without departing from the spirit or scope of this disclosure. All directional references (e.g., upper, lower, upward, downward, left, right, leftward, rightward, top, bottom, above, below, vertical, horizontal, clockwise, and counterclockwise) are only used for identification purposes to aid the reader's understanding of the present disclosure, and do not create limitations, particularly as to the position, orientation, or use of the disclosure. Joinder references (e.g., attached, coupled, connected, and the like) are to be construed broadly and may include intermediate members between a connection of elements and relative movement between elements. As such, joinder references do not necessarily infer that two elements are directly connected and in fixed relation to each other. It is intended that all matter contained in the above description or shown in the accompanying drawings shall be interpreted as illustrative only and not limiting. Changes in detail or structure may be made without departing from the spirit of the disclosure as defined in the appended claims.

When introducing elements of the present disclosure or the preferred embodiment(s) thereof, the articles "a", "an", "the", and "said" are intended to mean that there are one or more of the elements. The terms "comprising", "including", and "having" are intended to be inclusive and mean that there may be additional elements other than the listed elements.

As various changes could be made in the above constructions without departing from the scope of the disclosure, it is intended that all matter contained in the above description or shown in the accompanying drawings shall be interpreted as illustrative and not in a limiting sense.

Aspects of the disclosure are as set out in the following numbered clauses:
1. An electroporation catheter comprising:
   a shaft;
   a plurality of splines forming a basket around a distal portion of the shaft, wherein each spline comprises at least one energizable electrode; and
   a balloon positioned within the basket formed by the plurality of splines, the balloon being selectively inflatable, and wherein the balloon comprises at least one radiopaque marking.
2. The electroporation catheter of clause 1 wherein the radiopaque marking comprises an elastic radiopaque ink and the radiopaque marking is configured to expand or contract as the balloon expands or contracts.
3. The electroporation catheter of clause 1 or 2 wherein the radiopaque marking is asymmetric.
4. The electroporation catheter of any of clauses 1 to 3 wherein the radiopaque marking is applied to the inner diameter of the balloon.
5. The electroporation catheter of any of clauses 1 to 4 wherein the radiopaque marking is applied to the outer diameter of the balloon.
6. The electroporation catheter of any of clauses 1 to 5 wherein the radiopaque marking is embedded into the material of the balloon.
7. The electroporation catheter of any of clauses 1 to 6 wherein the balloon comprises at least two radiopaque markings.
8. The electroporation catheter of any of clauses 1 to 7 wherein the at least one radiopaque marking is applied by pad printing.
9. The electroporation catheter of any of clauses 1 to 8 wherein the at least one radiopaque marking is applied by painting.
10. The electroporation catheter of any of clauses 1 to 9 wherein the at least one radiopaque marking comprises a radiopaque ink applied during manufacturing.
11. The electroporation catheter of any of clauses 1 to 10 wherein each spline extends between a proximal end that is coupled to the shaft and distal end that is coupled to the shaft, and wherein the shaft comprises:
   an outer shaft element; and
   an inner shaft element extending through the outer shaft element, wherein the distal end of each spline is operatively connected to a distal end of the inner shaft element, wherein the proximal end of each spline is connected to a distal end of the outer shaft element, and wherein the inner shaft element is slidable relative to the outer shaft element to adjust an effective diameter of the basket formed by the plurality of splines.
12. The electroporation catheter of claim any of clauses 1 to 11 further comprising a valve for selectively deflating and inflating the balloon to occupy the space within the plurality of splines.
13. A balloon for an electroporation system, the balloon being selectively inflatable, the balloon comprising at least one radiopaque marking.
14. The balloon of clause 13 wherein the radiopaque marking comprises an elastic radiopaque ink and the radiopaque marking is configured to expand or contract as the balloon expands or contracts.
15. The balloon of clause 13 or 14 wherein the radiopaque marking is asymmetric.
16. The balloon of any of clauses 13 to 15 wherein the radiopaque marking is applied to the inner diameter of the balloon.
17. The balloon of any of clauses 13 to 16 wherein the radiopaque marking is applied to the outer diameter of the balloon.
18. The balloon of any of clauses 13 to 17 wherein the radiopaque marking is embedded into the material of the balloon.
19. The balloon of any of clauses 13 to 18 wherein the balloon comprises at least two radiopaque markings.
20. An electroporation system comprising:
   a generator; and
   a catheter coupled to the generator, the catheter comprising:
      a handle;
      a shaft extending distally from the handle;
      a plurality of splines forming a basket around a distal portion of the shaft, wherein each spline comprises at least one energizable electrode; and
      a balloon positioned within the basket formed by the plurality of splines, the balloon comprising at least one radiopaque marking.
21. A method of deploying an electroporation catheter assembly, the method comprising:
   delivering the electroporation catheter to a target site;
   deploying splines from the catheter to form a basket around a central shaft;
   inflating a balloon to occupy the space between the splines and conform to the shape of the basket; and
   locating the balloon using one or more radiopaque markers on the balloon.

## Claims

1. An electroporation catheter comprising:
a shaft;
a plurality of splines forming a basket around a distal portion of the shaft, wherein each spline comprises at least one energizable electrode; and
a balloon positioned within the basket formed by the plurality of splines, the balloon being selectively inflatable, and wherein the balloon comprises at least one radiopaque marking.

2. The electroporation catheter of claim 1 wherein the radiopaque marking comprises an elastic radiopaque ink and the radiopaque marking is configured to expand or contract as the balloon expands or contracts.

3. The electroporation catheter of claims 1 or 2 wherein the radiopaque marking is asymmetric.

4. The electroporation catheter of claims 1 to 3 wherein the radiopaque marking is applied to an inner diameter and/or an outer diameter of the balloon.

5. The electroporation catheter of claims 1 to 4 wherein the radiopaque marking is embedded into the material of the balloon.

6. The electroporation catheter of claims 1 to 5 wherein the balloon comprises at least two radiopaque markings.

7. The electroporation catheter of claims 1 to 6 wherein the at least one radiopaque marking is applied by pad printing and/or painting.

8. The electroporation catheter of claims 1 to 7 wherein each spline extends between a proximal end that is coupled to the shaft and distal end that is coupled to the shaft, and wherein the shaft comprises:
an outer shaft element; and
an inner shaft element extending through the outer shaft element, wherein the distal end of each spline is operatively connected to a distal end of the inner shaft element, wherein the proximal end of each spline is connected to a distal end of the outer shaft element, and wherein the inner shaft element is slidable relative to the outer shaft element to adjust an effective diameter of the basket formed by the plurality of splines.

9. The electroporation catheter of claims 1 to 10 further comprising a valve for selectively deflating and inflating the balloon to occupy the space within the plurality of splines.

10. A balloon for an electroporation system, the balloon being selectively inflatable, the balloon comprising at least one radiopaque marking.

11. The balloon of claim 10 wherein the radiopaque marking comprises an elastic radiopaque ink and the radiopaque marking is configured to expand or contract as the balloon expands or contracts.

12. The balloon of claims 10 or 11 wherein the radiopaque marking is asymmetric.

13. The balloon of claims 10 to 12 wherein the radiopaque marking is applied to an inner diameter and/or an outer diameter of the balloon.

14. The balloon of claims 10 to 13 wherein the radiopaque marking is embedded into the material of the balloon.

15. The balloon of claims 10 to 14 wherein the balloon comprises at least two radiopaque markings.
